# EUROPEAN PATENT APPLICATION

(11) **EP 3 671 724 A1**
(43) Date of publication of application: **24.06.2020**
(21) Application number: 18212948.6
(22) Date of filing: 17.12.2018
(51) Int. Cl.: G10H 1/00, H04S 7/00, A61M 21/00, G06F 3/16, A61B 5/16

(54) **PLAYBACK OF PERSONALISED AUDIO**

(71) Applicant: Nokia Technologies Oy, 02610 Espoo (FI)
(72) Inventor: VAN DEN BROECK, Marc, 2860 Sint-Katelijne-Waver (BE); FORLIVESI, Claudio, 1200 Woluwe-Saint-Lambert (BE); ACER, Utku Gunay, 2260 Hoboken (BE)
(74) Representative: Nokia EPO representatives

(57) **Abstract**

An apparatus is disclosed, comprising a means for detecting, based on data associated with a user, a current context of the user. The apparatus may also comprise a means for capturing audio content and associating it with the user, for subsequent playback responsive to the detecting means identifying the current context of the user corresponding to a first predetermined context. In some embodiments, the apparatus may comprise a means responsive to detecting the current context of the user corresponding to a second predetermined context, causing output of captured audio content associated with the user, the captured audio content having been previously captured based on the captured audio content being associated with a first predetermined context of the user.

## Description

### Field

Embodiments herein relate to methods and systems for playback of personalised audio.

### Background

It is known for played audio sounds to enhance and detract from a user's mood and/or effectiveness in performing certain tasks. For example, it is known to provide audio data representing generic recordings of white noise or background noise for playback as and when a user chooses.

### Summary

According to a first aspect, there is provided an apparatus, comprising: means for detecting, based on data associated with a user, a current context of the user; and means for capturing audio content and associating it with the user, for subsequent playback responsive to the detecting means identifying the current context of the user corresponding to a first predetermined context.

The detecting means may be configured to detect a current emotional and/or mental state of the user as the current context.

The audio content may represent ambient noise or sounds, e.g. noises or sounds that were generated in proximity to the user at the time of capture.

The capturing means may be configured automatically to cease capture of the audio content either upon detection by the detecting means of a context of the user that is different from the first predetermined context or after a predetermined capture period.

The apparatus may further comprise means for processing the captured audio content prior to subsequent playback to modify one or more audio characteristics.

The processing means may be configured to identify speech in the captured audio content and to modify data corresponding to said speech to remove it or make it unintelligible.

The processing means may be configured to identify high amplitude portions, above an allowable amplitude range, in the captured audio content and to modify said portions to remove them or to reduce their amplitude.

The processing means may be configured to modify said portions to reduce their amplitude in relation to the amplitude of other portions within the allowable amplitude range.

The apparatus may further comprise means for causing playback of the captured audio content responsive to detection by the detecting means of a second predetermined context of the user that is different from the first predetermined context.

One or more sets of captured audio content may be associated with respective positive contexts of the user, and wherein the means for causing playback may be configured to cause playback responsive to detection of the second predetermined context associated with a negative context of the first user.

The detecting means may be configured to determine the context of the user by receiving user data from one or more input devices for comparison with trained data particular to the user.

The one or more input devices may comprise one or more biometric sensors.

The one or more sensors may comprise body worn sensors, being one or more of a heart-rate sensor, a blood-pressure sensor, a movement sensor, a gesture sensor and a perspiration sensor.

The one or more input devices may comprise a keyboard device.

The apparatus may comprise an ear-worn device comprising one or more speakers. The ear-worn device may comprise a microphone for audio capture.

The capturing means may be configured automatically to capture the audio content, without user engagement.

According to a second aspect, there is provided a method comprising: detecting, based on data associated with a user, a current context of the user; and capturing audio content and associating it with the user, for subsequent playback responsive to the detecting means identifying the current context of the user corresponding to a first predetermined context.

According to a third aspect, there is provided an apparatus, comprising: means for detecting, based on data associated with a user, a current context of the user; means responsive to detecting the current context of the user corresponding to a first predetermined context, causing output of captured audio content associated with the user, the captured audio content having been previously captured based on the captured audio content being associated with a second predetermined context of the user.

According to a fourth aspect, there is provided a method comprising: detecting, based on data associated with a user, a current context of the user; responsive to detecting the current context of the user corresponding to a first predetermined context, causing output of captured audio content associated with the user, the captured audio content having been previously captured based on the captured audio content being associated with a second predetermined context of the user.

According to a fifth aspect, there is provided an apparatus, comprising: means for detecting, based on data associated with a user, a current context of the user; means for capturing audio content and associating it with the user based on the current context being a second predetermined context, for subsequent playback responsive to the detecting means identifying the context of the user corresponding to a first predetermined context; and means responsive to subsequently detecting the context of the user corresponding to the first predetermined context, to cause output of the captured audio content associated with the user corresponding to the second predetermined context of the user.

According to a sixth aspect, there is provided a computer program comprising instructions for causing an apparatus to perform: detecting, based on data associated with a user, a current context of the user; and capturing audio content and associating it with the user based on the current context being a second predetermined context, for subsequent playback responsive to the detecting means identifying the context of the user corresponding to a first predetermined context.

According to a seventh aspect, there is provided a computer program comprising instructions for causing an apparatus to perform: detecting, based on data associated with a user, a current context of the user; and responsive to detecting the context of the user corresponding to a first predetermined context, causing output of captured audio content associated with the user corresponding to a second predetermined context of the user.

According to an eighth aspect, there is provided a non-transitory computer readable medium comprising program instructions stored thereon for performing a method, comprising: detecting, based on data associated with a user, a current context of the user; and capturing audio content and associating it with the user, for subsequent playback responsive to the detecting means identifying the current context of the user corresponding to a first predetermined context.

According to a ninth aspect, there is provided an apparatus comprising: at least one processor; and at least one memory including computer program code which, when executed by the at least one processor, causes the apparatus: to detect, based on data associated with a user, a current context of the user; and to capture audio content and to associate it with the user, for subsequent playback responsive to the detecting means identifying the current context of the user corresponding to a first predetermined context.

According to a tenth aspect, there is provided a non-transitory computer readable medium comprising program instructions stored thereon for performing a method, comprising: detecting, based on data associated with a user, a current context of the user; and responsive to detecting the current context of the user corresponding to a first predetermined context, causing output of captured audio content associated with the user, the captured audio content having been captured based on the captured audio content being associated with a second predetermined context of the user.

According to an eleventh aspect, there is provided an apparatus comprising: at least one processor; and at least one memory including computer program code which, when executed by the at least one processor, causes the apparatus: to detecting, based on data associated with a user, a current context of the user; and, responsive to detecting the current context of the user corresponding to a first predetermined context, to cause output of captured audio content associated with the user, the captured audio content having been previously captured based on the captured audio content being associated with a second predetermined context of the user.

### Brief Description of Drawings

Example embodiments will now be described, by way of non-limiting example, with reference to the accompanying drawings, in which:
FIG. 1 is a schematic block diagram of a system for capture and playback of personalised audio, according to an example embodiment;
FIG. 2 is a schematic diagram of another system for capture and playback of personalised audio, shown in relation to a user, according to an example embodiment;;
FIG. 3 is a flow diagram showing processing operations which may be performed by systems according to some example embodiments;
FIG. 4 is a flow diagram showing processing operations of a training process, according to some example embodiments;
FIG. 5 is a schematic representation to enable understanding of how a positive and negative mood may be visualized with respect to baseline data, according to some example embodiments;
FIG. 6 is a flow diagram showing other processing operations which may be performed by systems according to some example embodiments;
FIG. 7 is a flow diagram showing audio content processing operations which may be performed by systems according to some example embodiments;
FIG. 8 shows hardware modules according to some example embodiments; and
FIG. 9 shows a non-volatile media according to some example embodiments.

### Detailed Description

Reference will now be made in detail to example embodiments for carrying out the subject matter of the present disclosure.

Example embodiments relate to audio capture and audio playback, which may be considered separately or in combination.

Example embodiments relate to determining a context of a user, that is some data-derivable way of determining circumstances relating to a user, for example for determining or estimating their mood. Example embodiments focus on mood as an example of context, but other applications may involve determining alternative or additional forms of context.

Example embodiments may involve playing back, through an output transducer such as a loudspeaker, headphones or an earphone, audio data which is personalised to a particular user for the purposes of changing their context, e.g. mood. The playback may be triggered automatically, responsive to detecting a relatively negative mood or a mood that is below a triggering threshold. The audio data, or at least audio data from which the played-back audio data is derived, may have been captured at an earlier time when it was determined that the user was in a more positive mood, e.g. above a capture triggering threshold. Thus example embodiments may also provide capturing audio data corresponding to ambient sounds in the vicinity of the user when the context of said user was determined to be positive. The audio data may therefore be considered personal to the user, as representing ambient sounds to the user when in the positive mood.

The concept of mood is not necessarily limited to an emotional state, i.e. happy or sad. It may also relate to other cognitive states, such as productive or non-productive, creative or non-creative, energetic or lethargic, etc. All are considered covered by the term mood for the purposes of this disclosure.

Regarding the meaning of positive and negative moods, a mood may be represented by the data, and one or more moods may be predetermined as being desirable and one or more other moods undesirable. A desirable mood may be considered a positive mood and an undesirable mood may be considered a negative mood.

Thus, embodiments provide a way of lifting a user's mood from a negative to a more positive mood based on ambient sounds, represented by the audio data, personalised to the user in the sense that a data-driven relationship between the ambient sounds and that user's mood has been established. The data-driven relationship may be a static one or may dynamically change over time, e.g. using machine learning to refine a learned model, such as a classification model.

Example embodiments may involve using one or more input devices, for example sensors, for providing data from which can be determined or estimated the mood of a user using an algorithm. The term "determined" as used herein may also cover "estimating" or "predicting" a mood. The sensors may comprise one or more biometric sensors, such as a heart rate sensor, a blood pressure monitor, a breathing monitor, a perspiration sensor, a gesture sensor, and/or any sensing means that measures some aspect of a live human body. Other examples may include image capturing sensors which may, for example, determine a user's mood based on facial expression, eye blinking rate, pupil dilation, etc. One or more data input devices may be provided from which can be deduced an indication of mood. For example, feedback from a computer input device such as a keyboard may be indicative of mood based on the number of keystrokes in a given time, the pattern of keystrokes, particular words that are entered, etc.

In some embodiments, a user's mood may be determined using data from just one input device, or alternatively, a combination of data from two or more input devices. Whichever input device or devices is or are used, the following description will use the term sensor data as being the data output by said device or devices.

The algorithm which takes said sensor data and determines a user's mood may determine a baseline at which the user is neither particularly negative nor positive, for example using sensed data when the user is at rest. The algorithm may thereafter determine whether the user is in a relatively positive or negative mood, from the baseline, based on the variation in data compared with that which was received when the baseline was determined. First and second offset thresholds may be provided either side of the baseline, such that the capturing and playback actions are performed when said thresholds are crossed, e.g. when the user is particularly positive or particularly negative. As mentioned previously, one or more moods may be predetermined as being desirable and one or more other moods undesirable. A desirable mood may be considered a positive mood and an undesirable mood may be considered a negative mood.

Alternatively, or additionally, the algorithm may request feedback from a user as to their current mood as part of a training process. For example, for an initial period, a user may be prompted through a user interface, e.g. on their smartphone, to indicate their current mood based on a scale of, e.g. one to ten or one to a hundred.

In some embodiments, the algorithm may involve interaction with a learned model providing classifications as to whether a mood is positive or negative based on a training stage. During use of the systems and methods to be described, the algorithm may update the learned model, for example based on whether a played back sound induces a relatively positive mood from the current mood, produces little or no change, or produces a more negative change. As will be known, feedback during an inference phase of a learned model may be used to update the model, for example by changing the value of weights between nodes of the learned model to refine the classification.

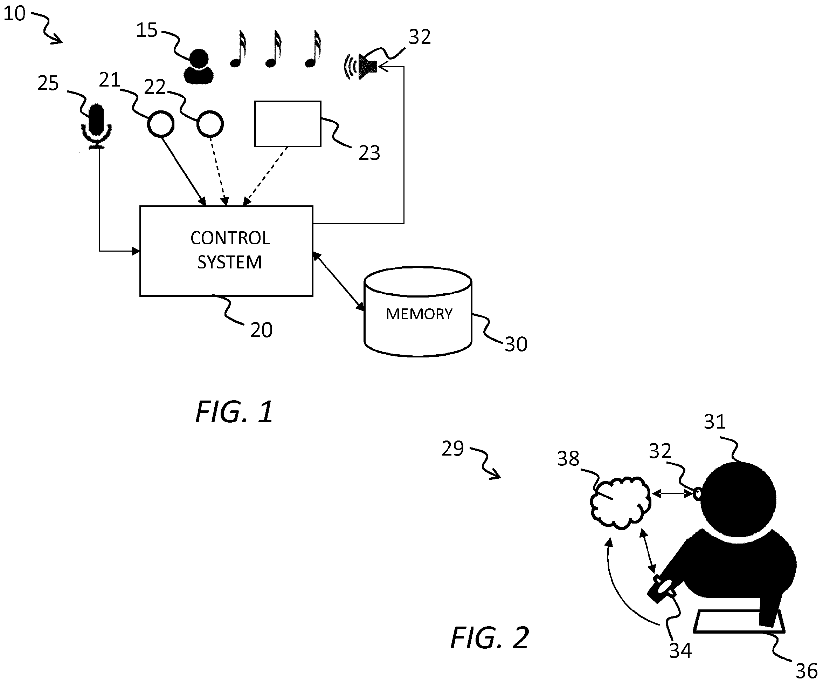

FIG. 1 shows an example system 10, in relation to a user 15, according to an example embodiment. The system 10 may comprise a control system 20, one or more sensors 21, 22, 23, an input transducer 25, a memory 30 and an output transducer 32. The input transducer 25 may comprise a microphone for converting audible sound into an electrical signal and the output transducer may comprise any transducer for converting an electrical or electronic signal into audible sound. As will be appreciated, in digital implementations, there will be an analogue-to-digital converter (ADC) and digital-to-analogue converter (DAC) respectively associated with the input and output transducers 25, 32. Typically, the output transducer 32 will comprise one or more loudspeakers, possibly embodied in an ear-worn device, such as a pair of headphones, a pair of earphones which locate partially within the ear canals or an earbud which locates partially within only one ear canal. In some embodiments, both the input and output transducers 25, 32 may be provided in a common module, such as within a pair of headphones, in an earphone or earbud, or within a larger module, such as a virtual reality (VR) headset.

In overview, the control system 20 may comprise one or more processors, although alternatively may comprise other hardware circuitry such as one or more microcontrollers, Application Specific Integrated Circuits (ASICS), Field Programmable Gate Arrays (FPGAs) and so on. The one or more processors, in conjunction with software code stored on non-transitory memory, may perform operations to be explained below. The control system 20 is configured to receive sensed data from the one or more sensors 21, 22, 23, to determine a current mood based on the sensed data, and to perform one or more actions based on the current mood. For example, if the current mood is determined to be positive, the control system 20 may initiate automatic capture of ambient sounds by means of the input transducer microphone 25 for possible processing and storage in the memory 30. For example, if the current mood is determined to be negative, the control system 20 may initiate automatic playback of the captured ambient sounds for that user through the output transducer 32, with the aim of inducing a more positive mood.

The memory 30 may comprise any suitable form of data memory and may comprise, for example, volatile memory, non-volatile memory, or some combination thereof. Although illustrated in FIG. 1 as a single memory, the memory 30 may comprise a plurality of memories. In various embodiments, the memory 30 may comprise, for example, a hard disk, random access memory, cache memory, flash memory, a compact disc read only memory (CD-ROM), digital versatile disc read only memory (DVD-ROM), an optical disc, circuitry configured to store information, or some combination thereof. The memory 30 may store one or more sets of audio data corresponding to ambient sounds personalised to a user and captured substantially at the time that user experienced a positive mood. The audio data may have associated metadata which may comprise one or more tags indicative of the positive association, and possibly a level of positivity, e.g. a number between one and ten or one and a hundred, with the higher number indicating a very positive mood and the lower number a reasonably positive mood.

In some embodiments, the memory 30 may store plural sets of audio data corresponding to different captured sounds for the user, each having been captured during a detected positive mood state. In some embodiments, the different sets of audio data may be played back in a predetermined order, e.g. in a loop, or randomly, or based on some other rule which may be based on context. For example, one or more sets of audio data may comprise metadata indicating a context (for example, the user's current environment, location, activity level) in which the respective set of audio data is played in preference to one or more other audio data sets, if a corresponding current context is determined. For example, the if the user is exercising, which may be detected using a heart rate and/or perspiration sensor, one of the audio sets may have associated metadata dictating that said audio set is to be played in that context. The different sets of audio data may be ranked based on their effectiveness, and possibly demoted or promoted based on feedback data during use of the system, e.g. dependent on whether a current sound induces a positive mood change. For example, an effectiveness score may be determined and updated based on feedback data. Feedback data may be received manually or automatically. Feedback data maybe solicited through a user interface, e.g. of the apparatus, a separate computer apparatus, and/or through voice prompts and voice commands to the apparatus. Additionally, or alternatively, feedback data may be based on sensor data which may represent a change in mood in response to the current sound. Positive feedback from the user may increase the effectiveness score and negative feedback may keep the effectiveness score static or reduce it.

As mentioned, the control system 20, one or more sensors 21, 22, 23, input transducer 25, memory 30 and output transducer 32 may be combined within a common system module. However, in some embodiments, some of said components may be in one or more separate system modules. For example, the control system 20 may be remote from the one or more sensors 21, 22, 23 which may be carried on one or more separate devices. For example, first and second sensors 21, 22 may be provided on a body-worn device such as a watch. For example, the third sensor 23 may comprise a keyboard for indicating a keystroke frequency. The one or more sensors 21, 22, 23 may therefore communicate their sensed data to the control system 20 via a network. For example, the input and output transducers 25, 32 may be separate devices and may be separate from the control system 20. Alternatively, the input and output transducers 25, 32 may be provided on a common system module but may be remote from the control system 20, with wireless communication transferring data between the elements. For example, the memory 30 may be cloud memory, remote from the other components; in such a case, the memory 30 should have a means for distinguishing users so as to determine which captured and personalised sounds belong to which users.

FIG. 2 shows another example system 29, in relation to a user 31. The system 29 comprises an earbud 32 which comprises the functionality of the control system 20, microphone input transducer 25, memory 30 and output transducer 32. A first sensor may be provided by a smartwatch 34 which determines at least heart rate and possibly blood pressure. The smartwatch 34 may also determine other sensed data, such as whether the user is currently engaged in a call and who the call is with, e.g. work or leisure related, which can contribute to mood. The sensed data from the smartwatch 34 may be relayed to the control system 20 in the earbud 32 by means of a network 38. Similarly, another sensor may be provided by a wireless keyboard 36, which either directly or via a computer terminal may relay information on keystrokes, words being typed etc. to the control system 20 in the earbud 32 by means of the network 38.

The network 38 may comprise any suitable wired or wireless network. For example, the network 38 may comprise a wireless network (for example, a cellular network, wireless local area network, wireless personal area network, wireless metropolitan area network, a Bluetooth connection and/or the like), a wireline network, or some combination thereof, and in some embodiments comprises at least a portion of the internet. In this regard, the network 38 may comprise any network configured for facilitating communication between the earbud 32 and one or more of the sensors 34, 36.

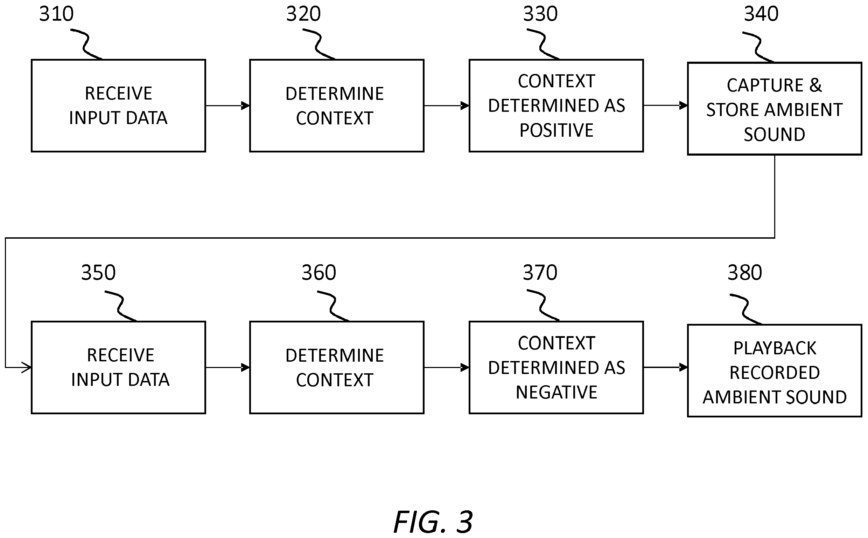

FIG. 3 is a flow diagram showing operations that may be performed by the control system 20 (possibly within the earbud 32 in the FIG. 2 example) described above. Some operations may be omitted and some other operations may be added. The numbering of operations is not necessarily indicative of the order of processing.

A first operation 310 may comprise receiving sensed input data from one or more sensors associated with a user.

A second operation 320 may comprise determining a current context, e.g. mood, of the user, based on the received input data.

A third operation 330 may comprise determining the context as a positive one.

A fourth operation 340 may comprise, responsive to the determination in the third operation 3.3, capturing and storing ambient sound. The capturing may commence without user engagement, that is it occurs automatically.

A fifth operation 350, performed at some point subsequent to the first to fourth operations 310 - 340, may comprise receiving further sensed input data from the one or more sensors associated with the user.

A sixth operation 360 may comprise determining the current context, e.g. mood, of the user, based on the received input data.

A seventh operation 370 may comprise, determining if the context is a negative one.

An eighth operation 380 may comprise, responsive to the determination in operation 370, playing back the recorded ambient sound captured in the fourth operation 340.

It will be appreciated that if, in the third operation 330, the context is determined as negative, then no capture of ambient sound may be performed. Similarly, if, in the seventh operation 370, the context is determined as positive, then no playback of the ambient sound may be performed. In this case, the process may automatically return to the third operation 330 if the measure of positivity corresponds to a capture action.

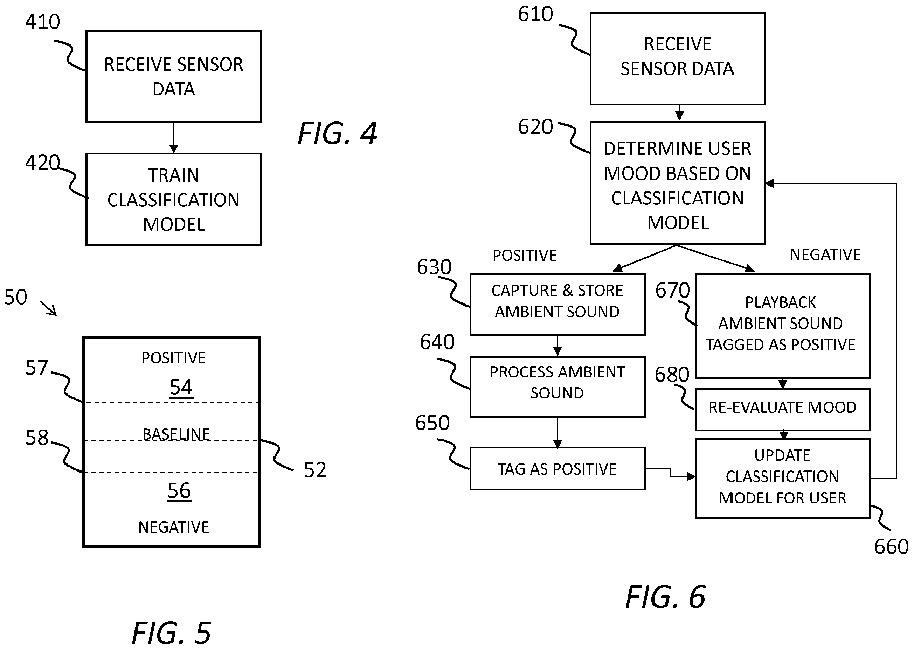

FIG. 4 is a flow diagram showing operations that may be performed by the control system 20 described above, where a learned classification model is employed. Some operations may be omitted and some other operations may be added. The numbering of operations is not necessarily indicative of the order of processing.

A first operation 410 may comprise receiving the sensor data from one or more sensors.

A second operation 420 may comprise training a classification model.

FIG. 5 is a schematic diagram showing graphically how a relatively positive and negative mood may be visualized with respect to baseline data 52. It will be referred to as a mood index 50, which may be reflected in the classification model. Where in the mood index 50 a user's current context lies may be inferred and updated to refine the classification model. The baseline data 52 may comprise a number or index determined using sensed data from the one or more sensors 21, 22, 23 when the user is neither particularly negative nor positive, for example when at rest. Alternatively, the baseline data 52 may be a default set of data provided in the classification model before use by the user.

Offset thresholds 57, 58 may be defined above and below which, respectively, the capture and playback actions are triggered. This avoids a constant switching between the two states when the user's mood is close to the baseline, and thereby saves power, processing and memory. The offset thresholds 57, 58 may be set by default, may be user-defined through an application, and/or may be dynamically updated.

Inferences may be drawn from variations in the sensed data subsequently. For example, with reference to FIG. 2, if a user's sensed heart rate and blood pressure sensed at the smartwatch 34 are raised from the baseline 52, and yet their keystroke input at the keyboard 32 is relatively low, or the keystrokes correspond to negative words or words predetermined as undesirable, such as "stressed", "sad", "miserable", then the data combined from the sensors may be mapped to the negative part 56 of the mood index 50. Conversely, if the user's sensed heart rate and blood pressure are raised and their keystroke input at the keyboard 32 is relatively high, or the keystrokes correspond to positive words or words predetermined as desirable, then a more positive or productive mood may be inferred. The data combined from the sensors may be mapped to the positive part 54 of the mood index 50.

Subsequently still, whether or not the resulting playback of sounds resulting from a negative mood determination actually promote the required more positive mood, can be used in re-evaluation of the classification model. For example, the baseline 52 may dynamically change based on feedback data and, where a learned model is used, the model may update responsive to the feedback data.

For example, FIG. 6 is a flow diagram showing operations that may be performed by the control system 20 described above. Some operations may be omitted and some other operations may be added. The numbering of operations is not necessarily indicative of the order of processing.

A first operation 610 may comprise receiving input data from one or more sensors associated with a user.

A second operation 620 may comprise determining a current context, e.g. mood, of the user, based on the received input data's mapping to a classification model.

A third operation 630 may comprise, responsive to determining the context as a positive one, initiating capture and storage of current ambient sounds.

A fourth operation 640 may comprise, optionally, processing the captured and stored ambient sounds, for example in accordance with methods to be described later on.

A fifth operation 650 may comprise tagging, e.g. marking in metadata associated with the stored ambient sounds, the stored audio data as positive.

A sixth operation 660 may comprise updating the classification model for the user based on the positive determination, i.e. to refine the model based on any changes in the sensed data from that currently stored in the classification model. The process may then return to the second operation 620 for subsequent sensed data.

A seventh operation 670, which may follow-on from the second operation 620, may comprise, responsive to determining the context as a negative one, initiating playback of a stored ambient sound tagged as positive for the user.

An eighth operation 680 may comprise re-evaluating the context, e.g. mood of the user to determine if it becomes more positive whilst the playback is ongoing. This may be performed periodically or continuously over a predetermined time period, e.g. 15 minutes.

Following the eighth operation 680, the sixth operation 660 may be returned to, whereby the classification model may be updated based on the re-evaluation. This may be to refine the model based on any changes in the sensed data from that currently stored in the classification model. Alternatively, or additionally, the update may demote the played-back sound because it is not having the desired impact on the user's mood. Demoting may involve promoting another set of audio data tagged as being positive for the user above the demoted one, such that the other set of data is played next time. Alternatively, or alternatively, it may trigger replacing the current set of audio data when a next capture event occurs.

As mentioned previously, for example in relation to the fourth operation 640, processing of the captured audio may be performed prior to storage and tagging. This processing may involve "cleaning up" the audio data to improve its positive qualities. It will be appreciated, however, that the fifth operation 650 (tagging) may be performed prior to the fourth operation 640.

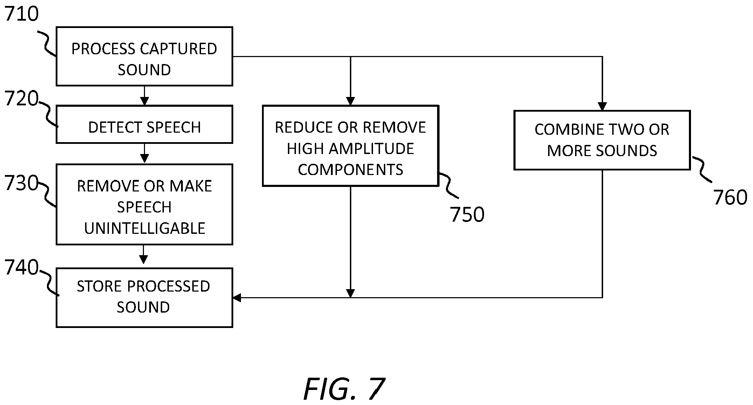

For example, FIG. 7 is a flow diagram showing operations that may be performed by the control system 20 described above. Some operations may be omitted and some other operations may be added. The numbering of operations is not necessarily indicative of the order of processing.

The operations shown in FIG. 7 relate to the processing of captured sounds 710, that is captured audio data in any of the above examples. Three example types of audio processing are mentioned, any of which can be used individually or in combination.

For example, one operation 720 may comprise detecting speech in the audio data, which can use any known speech detection technique. Responsive to detecting speech, in a further operation 730, processing may be performed to remove the speech or make it unintelligible, i.e. so that the speech cannot easily be understood (it might otherwise be distracting.) In a further operation 740, the processed speech may be stored.

For example, another operation 750 may comprise reducing or removing high amplitude components, i.e. components having an amplitude above a predetermined level or outside of an allowable range. In the operation 740, the processed sound may be stored. In some embodiments, the high amplitude components may be reduced in relation to the amplitude of other portions below the predetermined level or within the allowable amplitude range.

For example, another operation 760 may comprise combining two or more sets of audio data tagged as positive to produce a new set of audio data. Combining may comprise providing one set of audio data after another set of audio data, for example to extend the length of the audio data when played. In the operation 740, the processed sound may be stored.

Example embodiments recognize that users may react to different sounds differently. A particular sound may induce a positive mood for one person but a negative or benign mood for another person.

Example embodiments include an earbud 32 as one form of wearable device in which such a system and method may be implemented. However, the system and method may be implemented in other types of device, wearable or otherwise, including a smartwatch, virtual reality headset, wristband, etc.

In some embodiments, capturing of audio data may be automatically triggered. In other embodiments, an alert may be issued (either visual or audible) to signal to the user that their ambient audio is, or is about to be, captured, e.g. after a five second delay. In other embodiments, a prompt may be issued to a display device, enabling user selection as to whether or not to capture ambient audio.

In some embodiments, the capturing of audio data may automatically cease, either upon detection of a different context (e.g. positive to baseline) or after a predetermined capture period. This ensures that the captured audio data is of a manageable file size. The playback may involve playing back the audio data on a loop, or based on a detected context changing in an adverse direction, e.g. from negative to more negative.

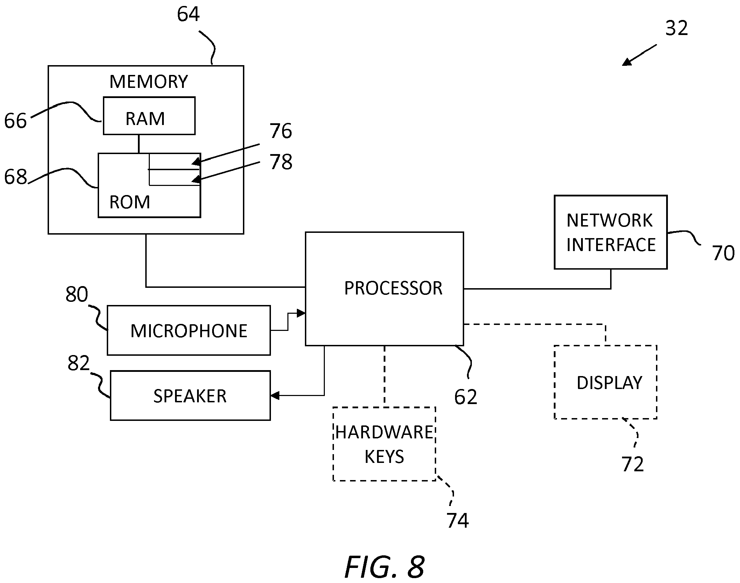

FIG. 8 is a schematic view of an apparatus 60 which may comprise the earbud 32 shown in FIG. 2.

The apparatus 60 may have a processor 62, a memory 64 closely-coupled to the processor and comprised of a RAM 66 and ROM 68. The apparatus 60 may comprise a network interface 70, and optionally a display 72 and one or more hardware keys 74. The apparatus 60 may comprise one or more such network interfaces 70 for connection to a network, e.g. a radio access network. The one or more network interfaces 70 may also be for connection to the internet, e.g. using WiFi or similar, such as 3G, LTE, 5G or other network protocols or future network protocols, including, for example, Bluetooth. The processor 62 is connected to each of the other components in order to control operation thereof. In some embodiments, the display 72 may comprise a touch-screen permitting user inputs and selections using the touch screen and/or by using a hovering gesture input. Alternatively, or additionally, the apparatus 60 may also comprise sensors such as one or more accelerometers and/or gyroscopes for individually or in combination sensing one or more user gestures, e.g. particular movements, which may serve as inputs in any of the above embodiments. Alternatively, or additionally, the apparatus 60 may comprise an audio input, e.g. a microphone, may be provided as a form of user input. The apparatus 60 may comprise a microphone input transducer 80 and a output transducer 82 such as a speaker.

The memory 64 may comprise a non-volatile memory, a hard disk drive (HDD) or a solid state drive (SSD). The ROM 68 of the memory stores, amongst other things, an operating system 76 and may store one or more software applications 78. The RAM 66 of the memory 64 may be used by the processor 62 for the temporary storage of data. The operating system 66 may contain code which, when executed by the processor, implements the operations as described above and also below, for example in the various flow diagrams of FIGS 3, 4, 6 and 7. As mentioned below, the memory 64 may comprise any suitable form, and may even be implemented in the cloud.

The processor 62 may take any suitable form. For instance, the processor 62 may be a microcontroller, plural microcontrollers, a processor, or plural processors and the processor may comprise processor circuitry.

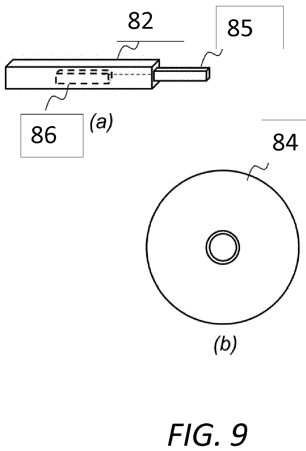

FIG. 9A and FIG. 9B show tangible non-volatile media, respectively a removable memory unit 82 and a compact disc (CD) 84, storing computer-readable code which when run by a computer may perform methods according to embodiments described above. The removable memory unit 82 may be a memory stick, e.g. a USB memory stick, having internal memory 86 storing the computer-readable code. The memory 86 may be accessed by a computer system via a connector 85. The CD 84 may be a CD-ROM or a DVD or similar. Other forms of tangible storage media may be used.

Implementations of any of the above described blocks, apparatuses, systems, techniques or methods include, as non-limiting examples, implementations as hardware, software, firmware, special purpose circuits or logic, general purpose hardware or controller or other computing devices, or some combination thereof. Some embodiments may be implemented in the cloud and utilize virtualized modules.

Embodiments of the present invention may be implemented in software, hardware, application logic or a combination of software, hardware and application logic. The software, application logic and/or hardware may reside on memory, or any computer media. In an example embodiment, the application logic, software or an instruction set is maintained on any one of various conventional computer-readable media. In the context of this document, a "memory" or "computer-readable medium" may be any non-transitory media or means that can contain, store, communicate, propagate or transport the instructions for use by or in connection with an instruction execution system, apparatus, or device, such as a computer.

Reference to, where relevant, "computer-readable storage medium", "computer program product", "tangibly embodied computer program" etc., or a "processor" or "processing circuitry" etc. should be understood to encompass not only computers having differing architectures such as single/multi-processor architectures and sequencers/parallel architectures, but also specialised circuits such as field programmable gate arrays FPGA, application specify circuits ASIC, signal processing devices and other devices. References to computer program, instructions, code etc. should be understood to express software for a programmable processor firmware such as the programmable content of a hardware device as instructions for a processor or configured or configuration settings for a fixed function device, gate array, programmable logic device, etc.

As used in this application, the term "circuitry" refers to all of the following: (a) hardware-only circuit implementations (such as implementations in only analogue and/or digital circuitry) and (b) to combinations of circuits and software (and/or firmware), such as (as applicable): (i) to a combination of processor(s) or (ii) to portions of processor(s)/software (including digital signal processor(s)), software, and memory(ies) that work together to cause an apparatus, such as a server, to perform various functions) and (c) to circuits, such as a microprocessor(s) or a portion of a microprocessor(s), that require software or firmware for operation, even if the software or firmware is not physically present.

In this brief description, reference has been made to various examples. The description of features or functions in relation to an example indicates that those features or functions are present in that example. The use of the term 'example' or 'for example' or 'may' in the text denotes, whether explicitly stated or not, that such features or functions are present in at least the described example, whether described as an example or not, and that they can be, but are not necessarily, present in some of or all other examples. Thus 'example', 'for example' or 'may' refers to a particular instance in a class of examples. A property of the instance can be a property of only that instance or a property of the class or a property of a sub-class of the class that includes some but not all of the instances in the class. It is therefore implicitly disclosed that a features described with reference to one example but not with reference to another example, can where possible be used in that other example but does not necessarily have to be used in that other example.

Although embodiments of the present invention have been described in the preceding paragraphs with reference to various examples, it should be appreciated that modifications to the examples given can be made without departing from the scope of the invention as claimed.

Features described in the preceding description may be used in combinations other than the combinations explicitly described.

Although functions have been described with reference to certain features, those functions may be performable by other features whether described or not.

Although features have been described with reference to certain embodiments, those features may also be present in other embodiments whether described or not.

Whilst endeavoring in the foregoing specification to draw attention to those features of the invention believed to be of particular importance it should be understood that the Applicant claims protection in respect of any patentable feature or combination of features hereinbefore referred to and/or shown in the drawings whether or not particular emphasis has been placed thereon.

## Claims

1. Apparatus, comprising:
means for detecting, based on data associated with a user, a current context of the user;
means for capturing audio content and associating it with the user, for subsequent playback responsive to the detecting means identifying the current context of the user corresponding to a first predetermined context.

2. The apparatus of claim 1, wherein the detecting means is configured to detect a current emotional and/or mental state of the user as the current context.

3. The apparatus of claim 1 or claim 2, wherein the capturing means is configured automatically to cease capture of the audio content either upon detection by the detecting means of a context of the user that is different from the first predetermined context or after a predetermined capture period.

4. The apparatus of any preceding claim, further comprising means for processing the captured audio content prior to subsequent playback to modify one or more audio characteristics.

5. The apparatus of claim 4, wherein the processing means is configured to identify speech in the captured audio content and to modify data corresponding to said speech to remove it or make it unintelligible

6. The apparatus of any preceding claim, further comprising means for causing playback of the captured audio content responsive to detection by the detecting means of a second predetermined context of the user that is different from the first predetermined context.

7. The apparatus of claim 6, wherein one or more sets of captured audio content is or are associated with respective positive contexts of the user, and wherein the means for causing playback is configured to cause playback responsive to detection of the second predetermined context associated with a negative context of the first user.

8. The apparatus of any preceding claim, wherein the detecting means is configured to determine the context of the user by receiving user data from one or more input devices for comparison with trained data particular to the user.

9. The apparatus of claim 8, wherein the one or more input devices comprise one or more biometric sensors.

10. The apparatus of claim 8 or claim 9, wherein the one or more sensors comprise body worn sensors, being one or more of a heart-rate sensor, a blood-pressure sensor, a movement sensor, a gesture sensor and a perspiration sensor.

11. The apparatus of any of claims 8 to 10, wherein the one or more input devices comprise a keyboard device.

12. The apparatus of any preceding claim, wherein the apparatus comprises an ear-worn device comprising one or more speakers.

13. The apparatus of any preceding claim, wherein the capturing means is configured automatically to capture the audio content, without user engagement.

14. A method comprising:
detecting, based on data associated with a user, a current context of the user;
capturing audio content and associating it with the user, for subsequent playback responsive to the detecting means identifying the current context of the user corresponding to a first predetermined context.

15. Apparatus, comprising:
means for detecting, based on data associated with a user, a current context of the user;
means responsive to detecting the current context of the user corresponding to a first predetermined context, causing output of captured audio content associated with the user, the captured audio content having been previously captured based on the captured audio content being associated with a second predetermined context of the user.

16. A method comprising:
detecting, based on data associated with a user, a current context of the user;
responsive to detecting the current context of the user corresponding to a first predetermined context, causing output of captured audio content associated with the user, the captured audio content having been previously captured based on the captured audio content being associated with a second predetermined context of the user.

17. An apparatus, comprising:
means for detecting, based on data associated with a user, a current context of the user;
means for capturing audio content and associating it with the user based on the current context being a second predetermined context, for subsequent playback responsive to the detecting means identifying the context of the user corresponding to a first predetermined context; and
means responsive to subsequently detecting the context of the user corresponding to the first predetermined context, to cause output of the captured audio content associated with the user corresponding to the second predetermined context of the user.

18. A computer program comprising instructions for causing an apparatus to perform:
detecting, based on data associated with a user, a current context of the user;
capturing audio content and associating it with the user based on the current context being a second predetermined context, for subsequent playback responsive to the detecting means identifying the context of the user corresponding to a first predetermined context, and/or
responsive to detecting the context of the user corresponding to a first predetermined context, causing output of captured audio content associated with the user corresponding to a second predetermined context of the user.
